# EUROPEAN PATENT SPECIFICATION

(11) **EP 2 825 640 B1**
(45) Date of publication and mention of the grant of the patent: **27.04.2016**
(21) Application number: 13708445.5
(22) Date of filing: 11.03.2013
(51) Int. Cl.: C12N 7/01, C12N 7/02, C12N 15/861

(54) **BATCHES OF RECOMBINANT ADENOVIRUS WITH ALTERED TERMINAL ENDS**
BATCHES VON REKOMBINANTEM ADENOVIRUS MIT VERÄNDERTEN GENOMISCHEN ENDSTÜCKEN
LOTS D'ADÉNOVIRUS RECOMBINANTS AVEC DES EXTRÉMITÉS GÉNOMIQUES TERMINALES ALTÉRÉES

(30) Priority: 12.03.2012 US 201261609678 P; 12.03.2012 EP 12159009
(43) Date of publication of application: 21.01.2015
(73) Proprietor: Crucell Holland B.V., 2333 CN Leiden (NL)
(72) Inventor: CUSTERS, Jerôme H.H.V., 2333 CN Leiden (NL); VELLINGA, Jort, 2333 CN Leiden (NL)
(74) Representative: Beslier, Victor
(86) International application number: PCT/EP2013/054846
(87) International publication number: WO 2013/135615

(56) References cited:
- WO-A2-2008/080003
- US-A1- 2009 227 000
- ROBERTS DIANE M ET AL: "Hexon-chimaeric adenovirus serotype 5 vectors circumvent pre-existing anti-vector immunity", NATURE: INTERNATIONAL WEEKLY JOURNAL OF SCIENCE, NATURE PUBLISHING GROUP, UNITED KINGDOM, vol. 441, no. 7090, 11 May 2006 (2006-05-11), pages 239-243, XP002385300, ISSN: 0028-0836, DOI: 10.1038/NATURE04721
- KINCHINGTON PAUL R ET AL: "Sequence changes in the human adenovirus type 5 DNA polymerase associated with resistance to the broad spectrum antiviral cidofovir", ANTIVIRAL RESEARCH, vol. 56, no. 1, October 2002 (2002-10), pages 73-84, XP002696552, ISSN: 0166-3542
- XIAOPING HE ET AL: "5/35 Fiber-Modified Conditionally Replicative Adenovirus Armed with p53 Shows Increased Tumor-Suppressing Capacity to Breast Cancer Cells", HUMAN GENE THERAPY, vol. 22, no. 3, 1 March 2011 (2011-03-01), pages 283-292, XP55061895, ISSN: 1043-0342, DOI: 10.1089/hum.2010.058
- H. KANEKO ET AL: "Analysis of the complete genome sequence of epidemic keratoconjunctivitis-related human adenovirus type 8, 19, 37 and a novel serotype", JOURNAL OF GENERAL VIROLOGY, vol. 90, no. 6, 1 June 2009 (2009-06-01), pages 1471-1476, XP055023766, ISSN: 0022-1317, DOI: 10.1099/vir.0.009225-0
- SILVER JIM ET AL: "Transduction and Oncolytic Profile of a Potent Replication-Competent Adenovirus 11p Vector (RCAd11pGFP) in Colon Carcinoma Cells", PLOS ONE, vol. 6, no. 3, March 2011 (2011-03), XP008162557, ISSN: 1932-6203
- XIAOPING HE ET AL: "5/35 Fiber-Modified Conditionally Replicative Adenovirus Armed with p53 Shows Increased Tumor-Suppressing Capacity to Breast Cancer Cells", HUMAN GENE THERAPY, vol. 22, no. 3, 1 March 2011 (2011-03-01), pages 283-292, XP055061895, ISSN: 1043-0342, DOI: 10.1089/hum.2010.058

### SEQUENCE LISTING

<110> Crucell Holland B.V.
   Vellinga, Jort
   Custers, Jerome
<120> Batches of recombinant adenovirus with altered terminal ends
<130> 0198 EP P00 PRI
<160> 14
<170> Patent In version 3.3
<210> 1
   <211> 17
   <212> DNA
   <213> Artificial
<220>
   <223> primer
<400> 1
   gtgactggtg agtactc 17
<210> 2
   <211> 16
   <212> DNA
   <213> Artificial
<220>
   <223> primer
<400> 2
   gaccacctag gctgac 16
<210> 3
   <211> 37
   <212> DNA
   <213> Artificial
<220>
   <223> primer
<400> 3
   ttaattaatc gatctatcta tataatatac cttatag 37
<210> 4
   <211> 36
   <212> DNA
   <213> Artificial
<220>
   <223> primer
<400> 4
   gatctatcta tataatatac cttatagatg gaatgg 36
<210> 5
<211> 35
   <212> DNA
   <213> Artificial
<220>
   <223> primer
<400> 5
   attatataga tagatcgatt aattaattcg aaccc 35
<210> 6
   <211> 18
   <212> DNA
   <213> Artificial
<220>
   <223> primer
<400> 6
   catcatcaat aatatacc 18
<210> 7
   <211> 18
   <212> DNA
   <213> Artificial
<220>
   <223> primer
<400> 7
   ctatctatat aatatacc 18
<210> 8
   <211> 22
   <212> DNA <213> Artificial
<220>
   <223> primer
<400> 8
   ctaagtagtt ccgtgagaaa ag 22
<210> 9
   <211> 20
   <212> DNA
   <213> Artificial
<220>
   <223> primer
<400> 9
   ggtacgtcac atcccattaa 20
<210> 10
   <211> 19
   <212> DNA
   <213> Artificial
<220>
   <223> primer
<400> 10
   cacttttgcc acatccgtc 19
<210> 11
   <211> 18
   <212> DNA
   <213> Artificial
<220>
   <223> primer
<400> 11
   cccacgttac gtcacttc 18
<210> 12
   <211> 17
   <212> DNA
   <213> Artificial
<220>
   <223> primer
<400> 12
   tgggcggtag gcgtgta 17
<210> 13
   <211> 22
   <212> DNA
   <213> Artificial
<220>
   <223> primer
<400> 13
   cgatctgacg gttcactaaa cg 22
<210> 14
   <211> 18
   <212> DNA
   <213> Artificial
<220>
   <223> primer
<400> 14
   tgggaggtct atataagc 18

## Description

The invention relates to the field of medicine and to the field of gene delivery for applications in vaccination and gene therapy. More in particular, the invention relates to batches of recombinant adenoviral vectors.

### Background of the invention

Recombinant human and animal adenoviruses are used extensively for their application in gene therapy and vaccination. The adenovirus vector is used as a carrier for a gene of interest to be introduced into host cells, for instance to express a gene or part thereof encoding a desired antigen to elicit an immune response.

More than 50 different human adenovirus serotypes have been identified. Of these, adenovirus serotype 5 (Ad5) has historically been studied most extensively for use as gene carrier. Recently, several other serotypes such as human Ad11, Ad26, Ad34, Ad35, Ad48, Ad49 and Ad50 and simian adenoviruses have been studied as vectors in view of lower levels of pre-existing neutralizing antibodies against these serotypes in the human population (see e.g. WO 00/70071). Promising examples of these are recombinant Ad35 (rAd35) and rAd26, which are studied in clinical trials.

The molecular biology of adenoviruses which possess a double stranded DNA genome of about 34-38 kb has been studied in detail. All adenoviruses are characterized by various inverted terminal repeats (ITRs) of about 100 bp in size (Dan et al., 2001, Virus Genes 22: 175-179; Liu et al., 2003, Curr Top Microbiol Immunol 272: 131-164), which are conserved among the serotypes of the different groups (Shinagawa et al., 1987, Gene 55: 85-93). The genome ends are covalently attached to the terminal protein (TP) at the 5' ends of the genome. The ITRs harbor the origin of replication (Bernstein et al., 1986, Mol Cell Biol 6: 2115-2124; Challberg & Rawlins, 1984, Proc Natl Acad Sci USA 81: 100-104; Guggenheimer et al., 1984, Proc Natl Acad Sci USA 81: 3069-3073; Harris & Hay, 1988, J Mol Biol 201: 57-67; Hay, 1985, EMBO J4: 421-426; van Bergen et al., 1983, Nucleic Acids Res 11: 1975-1989; Wang & Pearson, 1985, Nucleic Acids Res 13: 5173-5187) and are crucial for DNA replication, containing binding sites for cellular proteins that promote replication and facilitating panhandle formation. The ITR sequences possess a short highly conserved canonical "core region" that ranges from nucleotide 9-18 (Liu et al., *supra*). The terminal 8 nucleotides, preceding this core region, however, vary between adenovirus types and isolates (Alestrom et al., 1982, Gene 18: 193-197; Dan et al., *supra;* Jacobs et al., 2004, J Gen Virol 85: 3361-3366; Purkayastha et al., 2005, J Clin Microbiol 43: 3083-3094; Rademaker et al., 2006, J Gen Virol 87: 553-562; Shinagawa et al., 1987, *supra;* Shinagawa et al., 1983, Virology 125: 491-495; Shinagawa & Padmanabhan, 1980, Proc Natl Acad Sci USA 77: 3831-3835; Tokunaga et al., 1982, Gene 18: 329-334; Houng et al., 2006, J Clin Virol 35: 381-387). While most adenoviruses display the CATCATCA sequence in the terminal 8 nucleotides, several alternative sequences have been described.

The demand for recombinant adenoviruses is raising steeply in view of the variety of diseases that appear amenable for treatment or prophylaxis using these gene transfer vehicles, in combination with the large number of people affected by these diseases and ever increasing population world-wide.

For clinical batches that are intended for administration to humans, large-scale production of recombinant adenovirus (rAd) must be safe and efficacious, and comply with Good Manufacturing Practice (GMP) guidelines. One aspect important in this respect, is the homogeneity of such produced rAd batches.

It is now surprisingly reported herein that changes were found in the sequence of the eight most terminal bases on the 5' end of the genome from certain rAds, resulting in batches that display heterogeneity with respect to these sequences.

Therefore, a need remains for providing rAd batches on a large scale, which batches display improved homogeneity. The present invention provides such batches, as well as methods for obtaining them. In addition, the rAd in the batches of the instant invention displays improved replication in production processes.

### Summary of the invention

The invention provides a composition comprising a plurality of recombinant adenovirus particles, wherein the recombinant adenovirus is a recombinant human adenovirus of serotype 5, 11a, 26, 34, 35, 48, 49 or 50, or a recombinant simian adenovirus, characterized in that the genomes of essentially all adenovirus particles in said composition comprise as the 5' terminal nucleotides the nucleotide sequence: CTATCTAT.

The invention further provides a method for preparing a batch of (preferably at least 1 x 10⁷) recombinant adenovirus particles that have essentially all the same nucleotide sequence in the 5' termini of their genomes, comprising: a) performing a molecular cloning step to exchange the naturally occurring 5' termini of an adenovirus genome with altered 5' termini comprising as the terminal nucleotides the nucleotide sequence: CTATCTAT, b) propagating in host cells the recombinant adenovirus having the altered 5' termini, and c) harvesting the recombinant adenovirus to obtain a batch of recombinant adenovirus particles that essentially all comprise as the 5' terminal nucleotides of their genomes the nucleotide sequence: CTATCTAT.

The invention also provides a method for preparing a batch of recombinant adenovirus particles that have essentially all the same nucleotide sequence in the 5' termini of their genomes, comprising: a) performing a plaque purification of an adenovirus, wherein the recombinant adenovirus is a recombinant human adenovirus of serotype 5, 11a, 26, 34, 35, 48, 49 or 50, or a recombinant simian adenovirus, to isolate an adenovirus or recombinant adenovirus from a single plaque, wherein said adenovirus or recombinant adenovirus comprises as the 5' terminal nucleotides of its genome the nucleotide sequence: CTATCTAT, b) propagating in host cells a recombinant adenovirus obtained from the single plaque of step a), and c) harvesting the recombinant adenovirus to obtain a batch of recombinant adenovirus particles that essentially all comprise as the 5' terminal nucleotides of their genomes the nucleotide sequence: CTATCTAT.

In certain aspects, the recombinant adenovirus in the compositions or methods, is a recombinant human adenovirus, and is preferably not of human adenovirus serotype 3, 4, 7, 8, 9, 11p, 15, 21, 29, 37 or 53. In certain embodiments, the recombinant adenovirus in the compositions or methods of the invention, is a recombinant human adenovirus of serotype 5, 26, 35, 49 or 50 as defined by the claims. Preferably, the recombinant adenovirus is a recombinant human adenovirus of serotype 26 or 35 embodiments.

In certain embodiments, the recombinant adenovirus lacks at least a portion of the E1 region.

The recombinant adenovirus comprises a transgene.

In certain embodiments, the composition or batch of recombinant adenovirus comprises at least 1 x 10⁷, preferably at least 1 x 10⁸, preferably at least 1 x 10⁹, preferably at least 1 x 10¹⁰ recombinant adenovirus particles.

In certain embodiments, the step b) of the methods of the invention is performed in a bioreactor, preferably having a volume of between about 2 liter and 20000 liter.

In certain embodiments, the methods of the invention further comprise purifying the recombinant adenovirus.

In certain embodiments of the compositions or methods of the invention, the recombinant adenovirus is formulated into a pharmaceutical composition.

### Brief description of the Figures

FIG. 1. Emergence of alternative ITR sequences. For details, see example 3.
FIG. 2. replication kinetics of Ad35 and Ad5 vectors with alternative and original ITR sequences. For details, see example 5.

### Detailed description of the invention

It is described herein that a specific 5' terminal sequence (CTATCTAT) is surprisingly found after several passages of various recombinant adenoviruses that initially contained other terminal sequences, and that the presence of this sequence can contribute to improved adenovirus production:

The inventors put this surprising observation into practical use by constructing and/or including an active selection step for obtaining recombinant adenoviruses of serotypes that reportedly have a different terminal sequence in their wild-type genomes, with genomes comprising as the 5' terminal nucleotides the nucleotide sequence CTATCTAT.

The present invention therefore relates to a particular sequence (CTATCTAT) at the terminus of the recombinant adenoviral genome and the use thereof in the production of recombinant adenoviruses. This terminal sequence may be employed in any adenovirus serotype that does not contain this sequence at the 5' terminal end of its wild type genome.

In principle, the compositions (batches) of adenovirus according to the invention can contain the sequence CTATCTAT at 100% of their 5' terminal genome ends (since the starting adenovirus has been actively created and/or selected according to the invention). In view of some natural mutations that may occasionally and randomly occur in any biological system, the actual number might be slightly below 100%, although in preferable embodiments the amount of terminal sequences other than CTATCTAT is below the detection limit in the adenovirus batches according to the invention. Hence, according to the invention, essentially all of the adenoviral genomes in the compositions or batches of recombinant adenovirus particles comprise as the 5' terminal sequences the nucleotide sequence CTATCTAT. The term 'essentially all' as used herein refers to at least 90%, preferably at least 98%, more preferably at least 99%, still more preferably at least 99.9%, up to 100% (of the adenovirus particles in the composition). This can for instance be determined by methods such as PCR, which can easily detect 1 in 1000 particles, and in the compositions of recombinant adenoviral particles of the invention no adenoviruses with the original terminal sequences were detectable.

A 'batch' of adenovirus according to the invention means a composition that has been produced in one production run in a single production vessel, or alternatively it can refer to the plurality of adenovirus particles in a composition that is present in a single container (e.g., bioreactor, bag, flask, bottle, multi-dose vial, single-dose vial, syringe, etc). A batch of adenovirus according to the invention or a composition comprising adenovirus according to the invention preferably comprises at least 10⁷ recombinant adenoviral particles, and in certain embodiments comprises at least 10⁸, 10⁹, or 10¹⁰, adenoviral particles, ). A batch or composition may or may not comprise further relevant components besides the recombinant adenovirus.

The term 'recombinant' for an adenovirus, as used herein implicates that it has been modified by the hand of man, e.g. it has altered terminal ends actively cloned therein and/or it comprises a heterologous gene, i.e. it is not a naturally occurring wild type adenovirus.

Sequences herein are provided from 5' to 3' direction, as custom in the art.

An "adenovirus capsid protein" refers to a protein on the capsid of an adenovirus that is involved in determining the serotype and/or tropism of a particular adenovirus. Adenoviral capsid proteins typically include the fiber, penton and/or hexon proteins. An adenovirus of (or 'based upon') a certain serotype according to the invention typically comprises fiber, penton and/or hexon proteins of that certain serotype, and preferably comprises fiber, penton and hexon protein of that certain serotype. These proteins are typically encoded by the genome of the recombinant adenovirus. A recombinant adenovirus of a certain serotype may optionally comprise and/or encode other proteins from other adenovirus serotypes.

A recombinant adenovirus is 'based upon' an adenovirus as used herein, by derivation from the wild type, at least in sequence. This can be accomplished by molecular cloning, using the wild type genome or parts thereof as starting material. It is also possible to use the known sequence of a wild type adenovirus genome to generate (parts of) the genome de novo by DNA synthesis, which can be performed using routine procedures by service companies having business in the field of DNA synthesis and/or molecular cloning (e.g. GeneArt, GenScripts, Invitrogen, Eurofins). Thus, as non-limiting examples, a recombinant adenovirus that is not based upon human Ad4 is a recombinant adenovirus that does not comprise penton, hexon and fiber of human Ad4; a recombinant adenovirus that comprises hexon, penton and fiber of Ad35 is considered a recombinant adenovirus based upon Ad35, etc.

Due to the extensive research that has been performed on adenovirus serotypes and their genomic organisation, the person skilled in the art is aware of the boundaries of the ITRs in an adenoviral genome. The sequence CTATCTAT, is located in the recombinant adenoviruses according to the present invention at the utmost terminal ends of the genome. For instance, the upper strand of the left ITR of wt Ad5 starts with 5'-CATCATCA...-3' and that sequence is changed according to the invention to the preferred sequence 5'-CTATCTAT...-3'. The person skilled in the art is aware of the fact that at the right ITR, this sequence from 5' to 3' is located in the lower strand.

Changing the original (parental) sequence to the altered sequence of the invention may be carried out by different means, which means in itself are known and routine to those of skill in the art. Examples are direct PCR generation of the sequences, or sub-cloning from original adenoviral genomes that are identified to contain the specified sequence at their termini.

When the sequence of one terminus is changed, for instance by using molecular biology techniques in a plasmid/cosmid homologous recombination procedure (see e.g. WO 99/55132), while the other terminus remains unchanged, the resulting adenovirus will, during production and replication, copy the left or the right ITR, resulting in a mixed population with adenoviruses having only amended termini and adenoviruses with only non-amended termini (which as outlined herein, will evolve towards a population with more and more altered termini having terminal sequence CTATCTAT, if cultured and propagated *in vitro* because of the growth advantage conferred by this terminal sequence). It is preferred that a recombinant adenovirus according to the present invention comprises a genome that comprises the sequence CTATCTAT at both the left and right genome terminal ends.

The recombinant adenoviruses according to the invention thus comprise as the 5' terminal nucleotides of the genome the nucleotide sequence: CTATCTAT.

The vectors of the present invention are recombinant adenoviruses, also referred to as recombinant adenoviral vectors. The preparation of recombinant adenoviral vectors is well known in the art.

In certain embodiments, an adenoviral vector according to the invention is deficient in at least one essential gene function of the E1 region, e.g. the E1a region and/or the E1b region, of the adenoviral genome that is required for viral replication. In certain aspects an adenoviral vector is deficient in at least part of the non-essential E3 region. In certain aspects, the vector is deficient in at least one essential gene function of the E1 region and at least part of the non-essential E3 region. The adenoviral vector can be "multiply deficient," meaning that the adenoviral vector is deficient in one or more essential gene functions in each of two or more regions of the adenoviral genome. For example, the aforementioned E1-deficient or E1-, E3-deficient adenoviral vectors can be further deficient in at least one essential gene of the E4 region and/or at least one essential gene of the E2 region (e.g., the E2A region and/or E2B region).

Adenoviral vectors, methods for construction thereof and methods for propagating thereof, are well known in the art and are described in, for example, U.S. Pat. Nos. 5,559,099, 5,837,511, 5,846,782, 5,851,806, 5,994,106, 5,994,128, 5,965,541, 5,981,225, 6,040,174, 6,020,191, and 6,113,913, and Thomas Shenk, "Adenoviridae and their Replication", M. S. Horwitz, "Adenoviruses", Chapters 67 and 68, respectively, in Virology, B. N. Fields et al., eds., 3d ed., Raven Press, Ltd., . New York (1996), and other references mentioned herein. Typically, construction of adenoviral vectors involves the use of standard molecular biological techniques, such as those described in, for example, Sambrook et al., Molecular Cloreing, a Laboratory Manual, 2d ed., Cold Spring Harbor Press, Cold Spring Harbor, N.Y. (1989), Watson et al., Recombinant DNA, 2d ed., Scientific American Books (1992), and Ausubel et al., Current Protocols in Molecular Biology, Wiley Interscience Publishers, NY (1995), and other references mentioned herein.

An adenovirus according to the invention belongs to the family of the *Adenoviridae* and preferably is one that belongs to the genus *Mastadenovirus.* It can be a human adenovirus, but also an adenovirus that infects other species, including but not limited to a bovine adenovirus (e.g. bovine adenovirus 3, BAdV3), a canine adenovirus (e.g. CAdV2), a porcine adenovirus (e.g. PAdV3 or 5), or a simian adenovirus (which includes a monkey adenovirus and an ape adenovirus, such as a chimpanzee adenovirus). Preferably, the adenovirus is a human adenovirus (HAdV, or AdHu; in the present invention a human adenovirus is meant if referred to Ad without indication of species, e.g. the brief notation "Ad5" means the same as HAdV5, which is human adenovirus serotype 5) or a simian adenovirus such as chimpanzee adenovirus (ChAd, AdCh, or SAdV).

Preferably, the recombinant adenovirus of the invention is an adenovirus for which the wild type has been reported to have a different sequence (than CTATCTAT, e.g. the often occurring sequence CATCATCA) at the 5' terminal end. The reported or inferred 5' terminal 8 nucleotides of various adenovirus serotypes are depicted in Table I. US 2009/227000 reports an Ad11p having CTATCTAT at the 5'terminal end. Most advanced studies have been performed using human adenoviruses, and human adenoviruses are preferred according to certain aspects of the invention. In certain aspects, the recombinant adenovirus is based upon a human adenovirus, and is not based upon a human adenovirus serotype 3, 4, 7, 8, 9, 11p, 15, 21, 29, 37 or 53. The recombinant adenovirus may be based upon a human adenovirus serotype 1, 2, 5, 6, 10, 11 a, 12, 14, 16, 17, 18, 19, 22, 26, 28, 31, 34, 35, 36, 40, 41, 46, 48, 49, 50, 53, 54, 55, 56 or 57. More preferably, the recombinant adenovirus is based upon a human adenovirus serotype 5, 11a, 26, 34, 35, 48, 49 or 50. According to a particularly preferred example, an adenovirus is a human adenovirus of one of the serotypes 26, 35, 48, 49 or 50. An advantage of these serotypes is a low seroprevalence and/or low pre-existing neutralizing antibody titers in the human population. The most preferred serotypes for the recombinant adenovirus are human serotype 35 or human serotype 26, both of which are evaluated in clinical trials. Preparation of rAd26 vectors is described, for example, in WO 2007/104792 and in Abbink et al., (2007) Virol 81(9): 4654-63. Exemplary genome sequences of Ad26 are found in GenBank Accession EF 153474 and in SEQ ID NO:1 of WO 2007/104792. Preparation of rAd35 vectors is described, for example, in US Patent No. 7,270,811, in WO 00/70071, and in Vogels et al., (2003) J Virol 77(15): 8263-71. Exemplary genome sequences of Ad35 are found in GenBank Accession AC_000019 and in Fig. 6 of WO 00/70071.

Simian adenoviruses generally also have a low seroprevalence and/or low pre-existing neutralizing antibody titers in the human population, and a significant amount of work has been reported using chimpanzee adenovirus vectors (e.g. US6083716; WO 2005/071093; WO 2010/086189; WO 2010085984; Farina et al, 2001, J Viol 75: 11603-13; Cohen et al, 2002, J Gen Virol 83: 151-55; Kobinger et al, 2006, Virology 346: 394-401; Tatsis et al., 2007, Molecular Therapy 15: 608-17; see also review by Bangari and Mittal, 2006, Vaccine 24: 849-62; and review by Lasaro and Ertl, 2009, Mol Ther 17: 1333-39). Hence, in other preferred embodiments, the recombinant adenovirus according to the invention is based upon a simian adenovirus, e.g. a chimpanzee adenovirus. In certain embodiments, the recombinant adenovirus is based upon simian adenovirus type 1, 7, 8, 21, 22, 23, 24, 25, 26, 27.1, 28.1, 29, 30, 31.1, 32, 33, 34, 35.1, 36, 37.2, 39, 40.1, 41.1, 42.1, 43, 44, 45, 46, 48, 49, 50 or SA7P.

The sequences of most of the human and non-human adenoviruses mentioned above are known, and for others can be obtained using routine procedures.

A recombinant adenovirus according to the invention may be replication-competent or replication-deficient.

In certain embodiments, the adenovirus is replication deficient, e.g. because it contains a deletion in the E1 region of the genome. As known to the skilled person, in case of deletions of essential regions from the adenovirus genome, the functions encoded by these regions have to be provided in trans, preferably by the producer cell, i.e. when parts or whole of E1, E2 and/or E4 regions are deleted from the adenovirus, these have to be present in the producer cell, for instance integrated in the genome thereof, or in the form of so-called helper adenovirus or helper plasmids. The adenovirus may also have a deletion in the E3 region, which is dispensable for replication, and hence such a deletion does not have to be complemented.

A producer cell (sometimes also referred to in the art and herein as 'packaging cell' or 'complementing cell' or 'host cell') that can be used can be any producer cell wherein a desired adenovirus can be propagated. For example, the propagation of recombinant adenovirus vectors is done in producer cells that complement deficiencies in the adenovirus. Such producer cells preferably have in their genome at least an adenovirus E1 sequence, and thereby are capable of complementing recombinant adenoviruses with a deletion in the E1 region. Any E1-complementing producer cell can be used, such as human retina cells immortalized by E1, e.g. 911 or PER.C6 cells (see US patent 5,994,128), E1-transformed amniocytes (See EP patent 1230354), E1-transformed A549 cells (see e.g. WO 98/39411, US patent 5,891,690), GH329:HeLa (Gao et al, 2000, Human Gene Therapy 11: 213-219), 293, and the like. In certain aspects, the producer cells are for instance HEK293 cells, or PER.C6 cells, or 911 cells, or IT293SF cells, and the like.

For E1-deficient adenoviruses that are not derived from subgroup C or E adenoviruses, it is preferred to exchange the E4-orf6 coding sequence of the non-subgroup C or E adenovirus with the E4-orf6 of an adenovirus of subgroup C such as Ad5. This allows propagation of such adenoviruses in well known complementing cell lines that express the E1 genes of Ad5, such as for example 293 cells or PER.C6 cells (see, e.g. Havenga et al, 2006, J. Gen. Virol. 87: 2135-2143; WO 03/104467,

In alternative examples, there is no need to place a heterologous E4orf6 region (e.g. of Ad5) in the adenoviral vector, but instead the E1-deficient non-subgroup C or E vector is propagated in a cell line that expresses both E1 and a compatible E4orf6, e.g. the 293-ORF6 cell line that expresses both E1 and E4orf6 from Ad5 (see e.g. Brough et al, 1996, J Virol 70: 6497-501 describing the generation of the 293-ORF6 cells; Abrahamsen et al, 1997, J Virol 71: 8946-51 and Nan et al, 2003, Gene Therapy 10: 326-36 each describing generation of E1 deleted non-subgroup C adenoviral vectors using such a cell line).

Alternatively, a complementing cell that expresses E1 from the serotype that is to be propagated can be used (see e.g. WO 00/70071, WO 02/40665).

For subgroup B adenoviruses, such as Ad35, having a deletion in the E1 region, it is preferred to retain the 3' end of the E1B 55K open reading frame in the adenovirus, for instance the 166 bp directly upstream of the pIX open reading frame or a fragment comprising this such as a 243 bp fragment directly upstream of the pIX start codon (marked at the 5' end by a *Bsu*36I restriction site in the Ad35 genome), since this increases the stability of the adenovirus because the promoter of the pIX gene is partly residing in this area (see, e.g. Havenga et al, 2006, J. Gen. Virol. 87: 2135-2143; WO 2004/001032).

"Heterologous nucleic acid" (also referred to herein as 'transgene') in adenoviruses of the invention is nucleic acid that is not naturally present in the adenovirus. It is introduced into the adenovirus for instance by standard molecular biology techniques. It may encode a protein of interest or part thereof. It can for instance be cloned into a deleted E1 or E3 region of an adenoviral vector. A transgene is generally operably linked to expression control sequences. This can for instance be done by placing the nucleic acid encoding the transgene(s) under the control of a promoter. Further regulatory sequences may be added. Many promoters can be used for expression of a transgene(s), and are known to the skilled person. A non-limiting example of a suitable promoter for obtaining expression in eukaryotic cells is a CMV-promoter (US 5,385,839), e.g. the CMV immediate early promoter, for instance comprising nt. -735 to +95 from the CMV immediate early gene enhancer/promoter. A polyadenylation signal, for example the bovine growth hormone polyA signal (US 5,122,458), may be present behind the transgene(s).

In certain aspects, it may be desired to express more than one protein from a single adenovirus, and in such cases more coding sequences may be linked to form a single transcript from a single expression cassette or may be present in two separate expression cassettes cloned in different parts of the adenoviral genome.

The identity of the transgene is not material for the instant invention, which is suitable for adenoviruses comprising any transgene. Suitable transgenes are well known to the skilled person, and for instance may include transgene open reading frames, for instance open reading frames coding for polypeptides that have a therapeutic effect, e.g. for gene therapy purposes, or polypeptides against which an immune response is desired when the rAd vector is used for vaccination purposes. Particularly preferred heterologous nucleic acids are genes of interest encoding antigenic determinants towards which an immune response needs to be raised. Such antigenic determinants are also typically referred to as antigens. Any desired antigen can be encoded by the adenovirus vector. In typical examples, antigens are peptides, polypeptides or proteins from organisms that may cause a disease or condition. Therefore, in a further preferred aspect, said heterologous nucleic acid of interest encodes an immunogenic determinant. More preferably, said immunogenic determinant is an antigen from a bacterium, a virus, yeast or a parasite. The diseases caused by such organisms are generally referred to as 'infectious disease' (and are thus not limited to organisms that 'infect' but also include those that enter the host and cause a disease). So-called 'self-antigens', e.g. tumour antigens, also form part of the state of the art, and may be encoded by heterologous nucleic acids in the recombinant adenoviruses according to the present invention. Non-limiting examples from which the antigenic determinants (or antigens) are taken are malaria-causing organisms, such as *Plasmodium falciparum,* tuberculosis-causing organism such as *Mycobacterium tuberculosis,* yeasts, or viruses. In other preferred embodiments, antigens from viruses such as flaviviruses (e.g., West Nile Virus, Hepatitis C Virus, Japanese Encephalitis Virus, Dengue Virus), ebola virus, Human Immunodeficiency Virus (HIV), and Marburg virus may be used in compositions according to the present invention. In one example, said antigen is the CS protein or immunogenic part thereof from *P. falciparum* (for examples of adenovirus vectors encoding CS, see e.g. Havenga et al, 2006, J. Gen. Virol. 87: 2135-2143; Ophorst et al, 2007, Vaccine 25:1426-36; WO 2004/055187). In another aspect, the antigenic determinant is a protein of one antigen-, or a fusion protein of several antigens from *M. tuberculoses*, such as the Ag85A, Ag85B and/or the TB10.4 proteins or immunogenic part(s) thereof (see for the construction and production of such TB vaccine viruses e.g. WO 2006/053871). In yet another aspect, said antigenic determinant is a viral glycoprotein or immunogenic part thereof, such as GP from a filovirus, such as ebola virus or Marburg virus (e.g. Sullivan et al., (2003) Nature 424(6949): 681-684; Sullivan, et al., (2006) PLoS Med 3(6): e177; Geisbert et al., (2011) J Virol 85: 4222-4233). In yet further aspects, said immunogenic determinant is from an HIV protein such as gag, pol, env, nef, or variants thereof (for examples of adenovirus based HIV vaccines, see e.g. WO 2009/026183, WO 2010/096561, WO 2006/120034, WO 02/22080, WO 01/02607). In other examples, said antigenic determinant is a HA, NA, M, or NP protein, or immunogenic part of any of these, from influenza virus (e.g. Zhou et al, 2010, Mol Ther 18:2182-9; Hu et al, 2011, Virus Res 155: 156-62; review by Vemula and Mittal, 2010, Expert Opin Biol The 10: 1469-87). In other example the antigenic determinant is a HA protein or immunogenic part thereof from a measles virus (e.g. WO 2004/037294). In other aspects, the antigenic determinant is rabies virus glycoprotein (e.g. Zhou et al, 2006, Mol Ther 14: 662-672).

The invention also provides a method for preparing a batch of recombinant adenovirus particles that have essentially all identical nucleotide sequences in the 5' termini of their genomes, the method comprising: a) performing a molecular cloning step to exchange the naturally occurring 5' termini of an adenovirus genome with altered 5' termini comprising as the terminal nucleotides the nucleotide sequence: CTATCTAT, b) propagating in host cells the recombinant adenovirus having the altered 5' termini, and c) harvesting the recombinant adenovirus to obtain a batch of recombinant adenovirus particles that essentially all comprise as the 5' terminal nucleotides of their genomes the nucleotide sequence: CTATCTAT. In this preferred aspect, the 5' termini of the genomes are actively changed by molecular cloning techniques, which are as such well known and routine to the person skilled in the art of molecular biology. The identification of this advantageous CTATCTAT terminal sequence herein, renders this active step possible. This step is advantageous whenever any adenovirus having a different 5' terminal sequence (i.e. not CTATCTAT) is used as starting material or basis for generation of a (batch or composition of) recombinant adenovirus according to the invention, e.g. for any of the preferred serotypes of the invention as indicated herein. An advantage is the control and certainty that from the outset the desired CTATCTAT sequence is present in all genomes of the seed adenovirus for step b), and in view of the stability of this sequence as reported herein, the resulting batches of adenovirus in step c) will comprise adenoviral particles that essentially all have the same desired 5' terminal sequence.

However, as an alternative to the molecular cloning route, one could now also use the naturally induced variation and select for an adenovirus that has the altered sequence CTATCTAT at its terminus, to obtain the requisite starting material with stable 5' termini for propagation into batches of adenovirus at any desired scale. Thus, as an alternative a method for preparing a batch of recombinant adenovirus particles is provided that have essentially all identical nucleotide sequences in the 5' termini of their genomes, comprising: a) performing a plaque purification of an adenovirus, not being human adenovirus serotype 3, 4, 7, 8, 9, 11p, 15, 21, 29, 37 or 53 or a recombinant form thereof, to isolate an adenovirus or recombinant adenovirus from a single plaque, wherein said adenovirus or recombinant adenovirus comprises as the 5' terminal nucleotides of its genome the nucleotide sequence: CTATCTAT, b) propagating in host cells a recombinant adenovirus obtained from the single plaque of step a), and c) harvesting the recombinant adenovirus to obtain a batch of recombinant adenovirus particles that essentially all comprise as the 5' terminal nucleotides of their genomes the nucleotide sequence: CTATCTAT. Here, the active step is the preparation of a single plaque of (recombinant) adenovirus and testing/confirming that the genome thereof comprises at its 5' terminal end the desired sequence CTATCTAT. The skilled person will appreciate that step a) of this example may be performed with either an already recombinant adenovirus, or with still a wild type adenovirus isolate, wherein in the latter case prior to step b) a step is performed to obtain the recombinant adenovirus (e.g. by cloning, to introduce the transgene in the genome). A step of plaque purification to ensure that the starting material for further work is homogeneous and derived from a single isolate can be performed using entirely routine procedures for the skilled person in the field of adenovirus manipulation. Actively selecting for a (recombinant) adenovirus that comprises as the 5' terminal nucleotides of its genome the nucleotide sequence CTATCTAT had not been described before, and prior to the present invention this would not have made any sense either. To the contrary, the identification of this sequence would have been seen as an anomaly and the plaque would have been disposed of as having a genetic alteration prior to the instant invention. It is the merit of the instant invention to select for such (recombinant) adenovirus as starting material to ensure genetic stability, resulting in batches of recombinant adenovirus that essentially all comprise the same desired 5' terminal nucleotides in their genomes. The recombinant adenovirus of the invention has potentially improved replication characteristics.

A host cell according to the methods of the invention can be a packaging cell, which may complement for deficiencies in the recombinant adenoviral genome, e.g. E1. Steps b) and c) of the methods of the invention are standard and routine steps in the preparation of batches of recombinant adenovirus, well known to the skilled person.

In certain aspects, step b) of these methods is performed in a bioreactor, which may have a volume of between about 1 liter to about 20000 liter. This enables obtaining sufficient quantities of the desired adenovirus compositions for use at industrial scale. The term 'about' for numerical values as used in the present disclosure means the value ± 10%. In certain examples, the working volume is between 10L and 10000L, e.g. between 20L and 2000L. The working volume is the effective culture volume in the bioreactor. The volume of the bioreactor may be chosen by the skilled person depending on the actual demand. The present invention ensures that the final product will have the same terminal ends for essentially all adenovirus particles in batches so produced, i.e. be genetically homogeneous, which is desired for a pharmaceutical product.

Most large-scale suspension cultures are operated as batch or fed-batch processes because they are the most straightforward to operate and scale up. Nowadays, continuous processes based on perfusion principles are becoming more common and are also suitable (see e.g. WO 2010/060719, and WO 2011/098592, which describe suitable methods for obtaining and purifying large amounts of recombinant adenoviruses).

Producer cells are cultured to increase cell and virus numbers and/or virus titers. Culturing a cell is done to enable it to metabolize, and/or grow and/or divide and/or produce virus of interest according to the invention. This can be accomplished by methods as such well known to persons skilled in the art, and includes but is not limited to providing nutrients for the cell, for instance in the appropriate culture media. Suitable culture media are well known to the skilled person and can generally be obtained from commercial sources in large quantities, or custom-made according to standard protocols. Culturing can be done for instance in dishes, roller bottles or in bioreactors, using batch, fed-batch, continuous systems and the like. Suitable conditions for culturing cells are known (see e.g. Tissue Culture, Academic Press, Kruse and Paterson, editors (1973), and R.I. Freshney, Culture of animal cells: A manual of basic technique, fourth edition (Wiley-Liss Inc., 2000, ISBN 0-471-34889-9).

Typically, the adenovirus will be exposed to the appropriate producer cell in a culture, permitting uptake of the virus. Usually, the optimal agitation is between about 50 and 300 rpm, typically about 100-200, e.g. about 150, typical DO is 20-60%, e.g. 40%, the optimal pH is between 6.7 and 7.7, the optimal temperature between 30 and 39°C, e.g. 34-37°C, and the optimal MOI between 5 and 1000, e.g. about 50-300. Typically, adenovirus infects producer cells spontaneously, and bringing the producer cells into contact with rAd particles is sufficient for infection of the cells. Generally, an adenovirus seed stock is added to the culture to initiate infection, and subsequently the adenovirus propagates in the producer cells. This is all routine for the person skilled in the art. Such an adenovirus seed stock according to the invention comprises recombinant adenovirus particles wherein the genomes of essentially all adenovirus particles in said seed stock comprise as the 5' terminal nucleotides the sequence CTATCTAT.

After infection of an adenovirus, the virus replicates inside the cell and is thereby amplified, a process referred to herein as propagation of adenovirus. Adenovirus infection results finally in the lysis of the cells being infected. The lytic characteristics of adenovirus therefore permits two different modes of virus production. The first mode is harvesting virus prior to cell lysis, employing external factors to lyse the cells. The second mode is harvesting virus supernatant after (almost) complete cell lysis by the produced virus (see e.g. US patent 6,485,958, describing the harvesting of adenovirus without lysis of the host cells by an external factor). It is preferred to employ external factors to actively lyse the cells for harvesting the adenovirus.

Methods that can be used for active cell lysis are known to the person skilled in the art, and have for instance been discussed in WO 98/22588, p. 28-35. Useful methods in this respect are for example, freeze-thaw, solid shear, hypertonic and/or hypotonic lysis, liquid shear, sonication, high pressure extrusion, detergent lysis, combinations of the above, and the like. In one example, the cells are lysed using at least one detergent. Use of a detergent for lysis has the advantage that it is an easy method, and that it is easily scalable.

Detergents that can be used, and the way they are employed, are generally known to the person skilled in the art. Several examples are for instance discussed in WO 98/22588, p. 29-33. Detergents can include anionic, cationic, zwitterionic, and nonionic detergents. The concentration of the detergent may be varied, for instance within the range of about 0.1%-5% (w/w). In one aspect, the detergent used is Triton X-100.

Nuclease may be employed to remove contaminating, i.e. mostly from the producer cell, nucleic acids. Exemplary nucleases suitable for use in the present invention include Benzonase^{®}, Pulmozyme^{®}, or any other DNase and/or RNase commonly used within the art. In preferred examples, the nuclease is Benzonase^{®}, which rapidly hydrolyzes nucleic acids by hydrolyzing internal phosphodiester bonds between specific nucleotides, thereby reducing the viscosity of the cell lysate. Benzonase^{®} can be commercially obtained from Merck KGaA (code W214950). The concentration in which the nuclease is employed is preferably within the range of 1-100 units/ml. Alternatively, or in addition to nuclease treatment, it is also possible to selectively precipitate host cell DNA away from adenovirus preparations during adenovirus purification, using selective precipitating agents such as domiphen bromide (see e.g. US 7,326,555; Goerke et al., 2005, Biotechnology and bioengineering, Vol. 91: 12-21; WO 2011/045378; WO 2011/045381).

Methods for harvesting adenovirus from cultures of producer cells have been extensively described in WO 2005/080556.

In certain embodiments, the harvested adenovirus is further purified. Purification of the adenovirus can be performed in several steps comprising clarification, ultrafiltration, diafiltration or separation with chromatography as described in for instance WO 05/080556. Clarification may be done by a filtration step, removing cell debris and other impurities from the cell lysate. Ultrafiltration is used to concentrate the virus solution. Diafiltration, or buffer exchange, using ultrafilters is a way for removal and exchange of salts, sugars and the like. The person skilled in the art knows how to find the optimal conditions for each purification step. Also WO 98/22588, describes methods for the production and purification of adenoviral vectors. The methods comprise growing host cells, infecting the host cells with adenovirus, harvesting and lysing the host cells, concentrating the crude lysate, exchanging the buffer of the crude lysate, treating the lysate with nuclease, and further purifying the virus using chromatography.

Preferably, purification employs at least one chromatography step, as for instance discussed in WO 98/22588, p. 61-70. Many processes have been described for the further purification of adenoviruses, wherein chromatography steps are included in the process. The person skilled in the art will be aware of these processes, and can vary the exact way of employing chromatographic steps to optimize the process. It is for instance possible to purify adenoviruses by anion exchange chromatography steps, see for instance WO 2005/080556. Many other adenovirus purification methods have been described and are within the reach of the skilled person. Further methods for producing and purifying adenoviruses are disclosed in for example WO 00/32754, WO 04/020971, US 5,837,520, US 6,261,823, and WO 2006/108707.

For administering to humans, the invention may employ pharmaceutical compositions comprising the rAd and a pharmaceutically acceptable carrier or excipient. In the present context, the term "Pharmaceutically acceptable" means that the carrier or excipient, at the dosages and concentrations employed, will not cause any unwanted or harmful effects in the subjects to which they are administered. Such pharmaceutically acceptable carriers and excipients are well known in the art (see Remington's Pharmaceutical Sciences, 18th edition, A. R. Gennaro, Ed., Mack Publishing Company [1990]; Pharmaceutical Formulation Development of Peptides and Proteins, S. Frokjaer and L. Hovgaard, Eds., Taylor & Francis [2000]; and Handbook of Pharmaceutical Excipients, 3rd edition, A. Kibbe, Ed., Pharmaceutical Press [2000]). The purified rAd preferably is formulated and administered as a sterile solution although it is also possible to utilize lyophilized preparations. Sterile solutions are prepared by sterile filtration or by other methods known per se in the art. The solutions are then lyophilized or filled into pharmaceutical dosage containers. The pH of the solution generally is in the range of pH 3.0 to 9.5, e.g pH 5.0 to 7.5. The rAd typically is in a solution having a suitable buffer, and the solution of rAd may also contain a salt. Optionally stabilizing agent may be present, such as albumin. In certain aspects, detergent is added. In certain examples, rAd may be formulated into an injectable preparation. These formulations contain effective amounts of rAd, are either sterile liquid solutions, liquid suspensions or lyophilized versions and optionally contain stabilizers or excipients. An adenovirus vaccine can also be aerosolized for intranasal administration (see e.g. WO 2009/117134).

For instance adenovirus may be stored in the buffer that is also used for the Adenovirus World Standard (Hoganson et al, Development of a stable adenoviral vector formulation, Bioprocessing March 2002, p. 43-48): 20 mM Tris pH 8, 25 mM NaCl, 2.5% glycerol. Another useful formulation buffer suitable for administration to humans is 20 mM Tris, 2 mM MgCl₂, 25 mM NaCl, sucrose 10% w/v, polysorbate-80 0.02% w/v. Obviously, many other buffers can be used, and several examples of suitable formulations for the storage and for pharmaceutical administration of purified (adeno)virus preparations can for instance be found in European patent no. 0853660, US patent 6,225,289 and in international patent applications WO 99/41416, WO 99/12568, WO 00/29024, WO 01/66137, WO 03/049763, WO 03/078592, WO 03/061708.

In certain examples a composition comprising the adenovirus further comprises one or more adjuvants. Adjuvants are known in the art to further increase the immune response to an applied antigenic determinant, and pharmaceutical compositions comprising adenovirus and suitable adjuvants are for instance disclosed in WO 2007/110409. The terms "adjuvant" and "immune stimulant" are used interchangeably herein, and are defined as one or more substances that cause stimulation of the immune system. In this context, an adjuvant is used to enhance an immune response to the adenovirus vectors of the invention. Examples of suitable adjuvants include aluminium salts such as aluminium hydroxide and/or aluminium phosphate; oil-emulsion compositions (or oil-in-water compositions), including squalene-water emulsions, such as MF59 (see e.g. WO 90/14837); saponin formulations, such as for example QS21 and Immunostimulating Complexes (ISCOMS) (see e.g. US 5,057,540; WO 90/03184, WO 96/11711, WO 2004/004762, WO 2005/002620); bacterial or microbial derivatives, examples of which are monophosphoryl lipid A (MPL), 3-O-deacylated MPL (3dMPL), CpG-motif containing oligonucleotides, ADP-ribosylating bacterial toxins or mutants thereof, such as *E. coli* heat labile enterotoxin LT, cholera toxin CT, and the like. It is also possible to use vector-encoded adjuvant, e.g. by using heterologous nucleic acid that encodes a fusion of the oligomerization domain of C4-binding protein (C4bp) to the antigen of interest (e.g. Solabomi et al, 2008, Infect Immun 76: 3817-23). In certain aspects the compositions comprise aluminium as an adjuvant, e.g. in the form of aluminium hydroxide, aluminium phosphate, aluminium potassium phosphate, or combinations thereof, in concentrations of 0.05 - 5 mg, e.g. from 0.075-1.0 mg, of aluminium content per dose.

In other aspects, the compositions do not comprise adjuvants.

Adenovirus compositions may be administered to a subject, e.g. a human subject. The total dose of the adenovirus provided to a subject during one administration can be varied as is known to the skilled practitioner, and is generally between 1x10⁷ viral particles (vp) and 1x10¹² vp, preferably between 1x10⁸ vp and 1x10¹¹ vp, for instance between 3x10⁸ and 5x10¹⁰ vp, for instance between 10⁹ and 3x10¹⁰ vp.

Administration of adenovirus compositions can be performed using standard routes of administration. Non-limiting aspects include parenteral administration, such as by injection, e.g. intradermal, intramuscular, etc, or subcutaneous or transcutaneous, or mucosal administration, e.g. intranasal, oral, and the like. In one example a composition is administered by intramuscular injection, e.g. into the deltoid muscle of the arm, or vastus lateralis muscle of the thigh. The skilled person knows the various possibilities to administer a composition, e.g. a vaccine in order to induce an immune response to the antigen(s) in the vaccine.

A subject as used herein preferably is a mammal, for instance a rodent, e.g. a mouse, or a non-human-primate, or a human. Preferably, the subject is a human subject.

It is also possible to provide one or more booster administrations of one or more adenovirus vaccines. If a boosting vaccination is performed, typically, such a boosting vaccination will be administered to the same subject at a moment between one week and one year, preferably between two weeks and four months, after administering the composition to the subject for the first time (which is in such cases referred to as 'priming vaccination'). In alternative boosting regimens, it is also possible to administer different vectors, e.g. one or more adenoviruses of different serotype, or other vectors such as MVA, or DNA, or protein, to the subject as a priming or boosting vaccination.

The invention is further explained in the following examples. They serve to clarify the invention.

### EXAMPLES

### Methods

### Plasmids:

The alternative ITR sequence was introduced into the left ITR by cloning into pAdapt and into the right ITR by cloning into pBr plasmids for Ad35 and Ad5, respectively (see e.g. Havenga M. et al, 2006, J. Gen. Virol. 87: 2135-2143; Havenga M. et al, 2001, J. Virol. 75: 3335-3342). To introduce the alternative ITR sequence into the left ITR, a fusion PCR was performed using a forward primer containing a ScaI site (GTGACTGGTGAGTACTC [SEQ ID NO: 1]), a reverse primer containing an AvrII site (GACCACCTAGGCTGAC [SEQ ID NO: 2]) and fusion forward and reverse primers harboring the alternative ITR sequence (alt ITR for 1: TTAATTAATCGAT***CTATCTATAT***AATATACCTTATAG [SEQ ID NO: 3], alt ITR for 2: GAT***CTATCTATAT***AATATACCTTATAGATGGAATGG [SEQ ID NO: 4], alt ITR rev: ATT***ATATAGATAG***ATCGATTAATTAATTCGAACCC [SEQ ID NO: 5]). Two partly overlapping ITR forward primers were used in the PCR for one of the fusion PCR fragments to increase the PCR efficiency on an extremely AT rich region in the template. The fusion PCR product was first subcloned into the pTopo vector to facilitate sucloning and then inserted into pAdapt35 plasmid via the AvrII and ScaI sites with the indicated transgenes.

To introduce the alternative ITR sequence into the right ITR, a fusion PCR was performed using a forward primer containing an NdeI site and a reverse primer containing an NruI site and fusion forward and reverse primers harboring the alternative ITR sequence using the same fusion PCR strategy as described for the left ITR. The fusion, PCR product was then subcloned into pTopo and subsequently into pBR.Ad35.PR.dE3 orf6/7 plasmid using NdeI and NruI.

To generate Ad5 vectors with alternative ITRs the same strategy as described above was used.

### Cell culture:

PER.C6 cells (Fallaux et al., 1998) were maintained in Dulbecco's modified Eagle's medium (DMEM) with 10% fetal bovine serum (FBS), supplemented with 10mM MgCl₂. A549, HEK293, Hep2, HeLa and MRC5 cells were obtained from ATCC and maintained in DMEM with 10% FBS.

### Adenovirus generation, infections and passaging:

If not otherwise stated, all viruses were generated in PER.C6 by single or double homologous recombination and produced as previously described (Havenga et al., 2006). Briefly, plasmids were transfected in PER.C6 using Lipofectamine according to the instructions provided by the manufacturer (Life Technologies). Cells were harvested one day after full CPE, freeze-thawed, centrifuged for 5 min at 3,000 rpm, and stored at -20°C. Of the crude lysate, 3 to 5 ml was used to inoculate 4×T175 triple-layer flasks containing 70% confluent layers of PER.C6 cells. The virus was purified using a two-step CsCl purification method. Finally, the virus was stored in aliquots at -85°C.

To investigate the switch from the original to an alternative ITR sequence, the different viruses were passaged serially using either crude virus material after plaque purification or purified virus batches as described above. To this end, cells were infected with the respective viral vector. One day after full CPE, the cells and the supernatant were harvested and frozen. The viral particles were released from the cells by thawing and this crude virus material was used to infect new cells.

### Viral DNA isolation from infected cells:

DNA isolations for the ITR-specific PCR were performed as follows. Viral particles were released from crude virus material by repeated freeze-thaw cycles. Afterwards, host cell DNA was removed by DNAse I treatment. Viral particles were disrupted by Incubation with 10% SDS and treated with proteinase K. Viral DNA was subsequently purified using the GeneClean Spin Kit (MP Biochemicals) and used for PCR analysis.

Crude lysate was used to isolate DNA for ITR sequence analysis. For this purpose, DNA was isolated by PEG isolation from 20 ml of crude cell lysate, lysed by consecutive freeze-thaw cycles and treated with DNAse I (0.01mg/ml Roche) and Rnase T1 (10U/ml Roche), followed by NaCl inactivation (1M). Viral particles were precipitated using 10% PEG 6000 (BDH iochemical) on ice for 1h, followed by a centrifugation step at 9000xg and resuspended in 1ml of SM buffer (0.1M NaCl, 8mM MgSO4, 50mM Tris HCl pH 7.5, 0.002% gelatine). Viral capsid proteins were disrupted using 10% SDS and proteinase K treatment and the DNA was extracted by phenol-chloroform precipitation. Full length DNA was digested by EcoRI (Ad26), SphI (Ad48, Ad5), AgeI (Ad49, Ad11), NheI (Ad50) and finally sequenced by Baseclear, Leiden.

### ITR-specific PCR

Since the ITR regions are AT rich, locked nucleic acid (LNA) primers were used to assure sufficient primer binding to the template. Primers were purchased from Eurogentech. The following primers were used. Lower case letter indicate LNA nucleotides. ori.ITR: CatcaTcaATAATATACC [SEQ ID NO: 6], Ad35 alt ITR: CtatcTatATAATATACC [SEQ ID NO: 7], Ad35 left ITR rev: CTAAGTAGTTCCGTGAGAAAAG [SEQ ID NO: 8]. Ad35 right ITR forward: GGTACGTCACATCCCATTAA [SEQ ID NO: 9], Ad5 left ITR rev: CACTTTTGCCACATCCGTC [SEQ ID NO: 10], Ad5 right ITR for: CCCACGTTACGTCACTTC [SEQ ID NO: 11]. PCR products were analyzed on an agarose gel.

### Replication kinetics by qPCR

Replication kinetics were analyzed by infection of 293 and PER.C6 cells using 1000 VP/cell for 3hours and subsequently washed. Presence of viral particles in cells and supernatant were analyzed at indicated time points post infection by a VP qPCR. To this end, infected cells were lysed using 0.5% Triton X-100 (Sigma), incubated at -80 degrees for 1 hour and thawed.

A qPCR specific for the CMV promoter, present in all used adenoviral vectors was performed using gene expression master mix (Applied Biosystems) according to manufacturer's recommendations. Primer/ probe combination sequences are: CMV for: TGGGCGGTAGGCGTGTA [SEQ ID NO: 12], CMV rev: CGATCTGACGGTTCACTAAACG [SEQ ID NO: 13], Probe 5'-VIC-TGGGAGGTCTATATAAGC-MGB-NFQ-3' [SEQ ID NO: 14], purchased from Applied Biosystems. To determine the amount of viral particles in the individual samples, a standard curve was generated.

### Sequence alignments

Adenovirus ITR sequences were obtained from BLAST search. The alignment was created using CLC software. Alignments are based on published sequences. However, for some of the published sequences, the ITRs have not specifically been sequenced. Instead, conservation across subtypes was assumed, which might lead to an overrepresentation of the conserved CATCATCA sequence. In case several sequences for one adenovirus serotype were published, they were only included if they differed from each other in the terminal 8 nucleotides.

### Example 1. Detection of an alternative ITR sequence during production of an Ad35 vaccine vector on PER. C6 cells

For generation of an Ad35 vaccine vector expressing *Mycobacterium tuberculosis* antigens Ag85A, Ag85B, and TB10.4 antigens as previously described (WO 2006/053871; (Radosevic et al., 2007, Infect. Immun. 75: 4105-4115), PER.C6 cells were transfected with linearized plasmids, yielding the Ad35.TBS virus, capable of replication in PER.C6 cells.

Prior to production, two consecutive plaque purifications ensure derivation of the virus seed from a single genetically stable clone. The obtained virus was characterized by identity PCR and Western Blot at different stages of the production process and completely sequenced before usage as a seed virus for large-scale production.

The genome sequence was stable and thus identical to the genome encoded by the rescue plasmids with the exception of the terminal 8 nucleotides at the left and the right ITR. The plasmid encoded sequence CATCATCA, named original ITR sequence in the following, switched to the sequence CTATCTAT, termed alternative ITR sequence, resulting in 6 nucleotide changes in comparison to the plasmid sequence. This finding was surprising since adenoviral genomes are considered to be highly stable thereby contributing to their suitability as vaccine vectors.

To investigate the inconsistency in the terminal ITR sequence further, we sequenced the ITRs at different steps during the production of the vaccine vector: This analysis revealed that the original ITR sequence was still present at five passages after plaque purification (VPN 5). However, we also detected a sequence with subpeaks indicating a mixing sequence at passage number 5 of a different production process. Except for the subpeaks within the terminal 8 nucleotides, the remaining sequence did not display any inconsistencies. At VPN 6 the sequence is mixed, likely being composed of approximately the same proportion of the original and the alternative sequence and turning into a distinct alternative sequence at VPN 7.

### Example 2. ITR heterogeneity occurs for different Ad35 vectors as well as for wildtype virus

To address whether the observed phenomenon is a negligible event and to examine the frequency of the switch from the original CATCATCA to the alternative CTATCTAT ITR sequence, we analyzed four plaques originating from the same virus rescue. Viruses propagated from all plaques switched to an alternative ITR sequence upon repeated passaging.

Furthermore, alternative ITRs were observed during passaging of Ad35 vectors expressing different transgenes and independent of a partial deletion of the pIX promoter (Table II).

Furthermore, we do not only see mixing sequences for Ad35 based vectors, but also for the Ad35 wildtype virus, excluding a vector artifact.

### Example 3. The Alternative ITR sequence is stable in Ad35.TBS over 10 viral passages

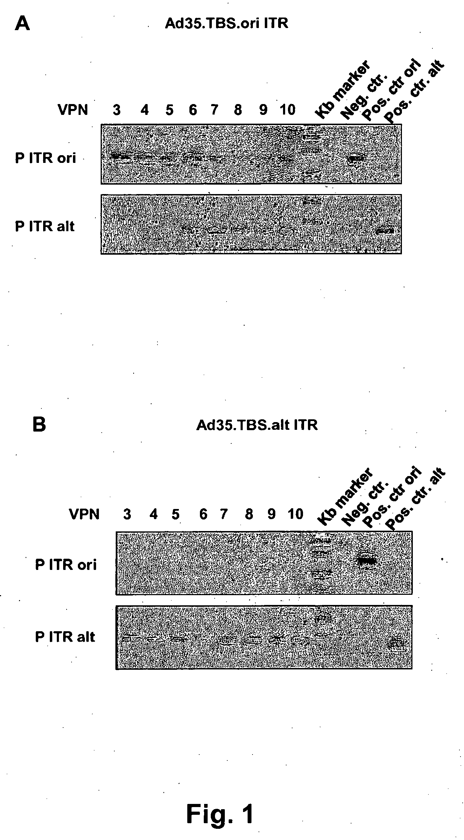

To address whether the switch to the alternative ITR sequence is stable over several viral passages, we constructed an Ad35.TBS harboring either original or alternative ITR sequences, termed Ad35.TBS.ori ITR and Ad35.TBS.alt ITR. These viruses were subjected to passaging in PER.C6 cells and the sequence of the 8 terminal ITR nucleotides was monitored by PCR analysis of each viral passage number. To distinguish the original from the alternative sequence, different PCR primer sets that specifically amplified either the original or the alternative ITR sequence were utilized. Analysis of each viral passage identified a decrease of the original sequence between VPN 3 and VPN6 and the emergence of the alternative sequence at VPN6 for passaging of Ad35.TBS.ori ITR (Fig. 1A). The alternative sequence was remained over 4 passages (Fig. 1A). During the passaging of artificially created Ad35.TBS.alt, only the alternative PCR sequence was detectable over 10 viral passages (Fig. 1B), excluding reversion to the original sequence or general instability within this part of the genome.

Furthermore, mixing of Ad35 vectors with alternative or original ITR sequences, which were otherwise identical, also led to outgrowth of the alternative ITR sequence, indicating a growth advantage of the alternative over the original ITR sequence.

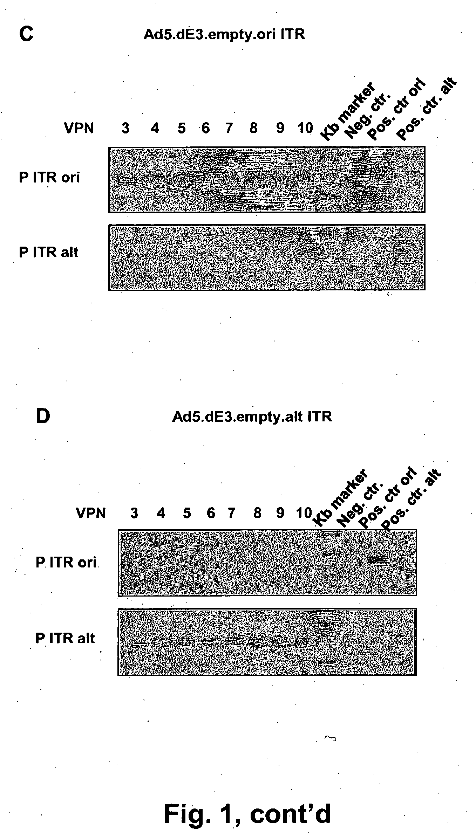

Since we detected the switch from the original ITR sequence to an alternative ITR sequence in Ad35, a group B vector, we additionally analyzed Ad5.empty.ori ITR and Ad35.empty.alt ITR, a group C vector harboring either original or alternative ITR sequences. In contrast to the results with the Ad35 vector, Ad5 did not display a switch in the ITR sequence, but retained the original ITRs over 10 viral passages (Fig. 1C) (however see example 8 below, showing that the alternative sequence was also found in Ad5 upon further passaging). Moreover, artificially generated Ad5 harboring alternative ITRs was stable over 10 viral passages and did not revert to the original ITR sequence (Fig. 1D).

### Example 4. Ad35 harboring the Alternative ITR sequence induces CPE at an earlier time point post infection than Ad35. ori ITR

Since we observed outgrowth of virus genomes with alternative ITRs, we assumed that viruses with alternative ITRs should have a replication advantage over those with original ITRs for Ad35. To test this, we used Ad35 viruses harboring either original or alternative ITR sequences and analyzed their growth kinetics. Since the CPE induced by adenoviral infection on E1-complementing cell lines is a good indication for replication speed, we first infected 293 cells and looked at the cytopathic effect at 24h, 48h, 72h and 96h post infection (hpi) at an MOI of 100 VP/cell and 1000 VP/cell. At 24h post infection, no CPE was observed for both 100 and 1000 VP/cell. However, at 48 hpi advanced CPE is observed for Ad35.dE1.alt ITRs at both 100 and 1000 VP/cell, developing into full CPE at 96hpi. By contrast, only limited CPE was seen for Ad35.dE1.ori ITR at these time points post infection.

### Example 5. The alternative ITR sequence confers a genome replication advantage

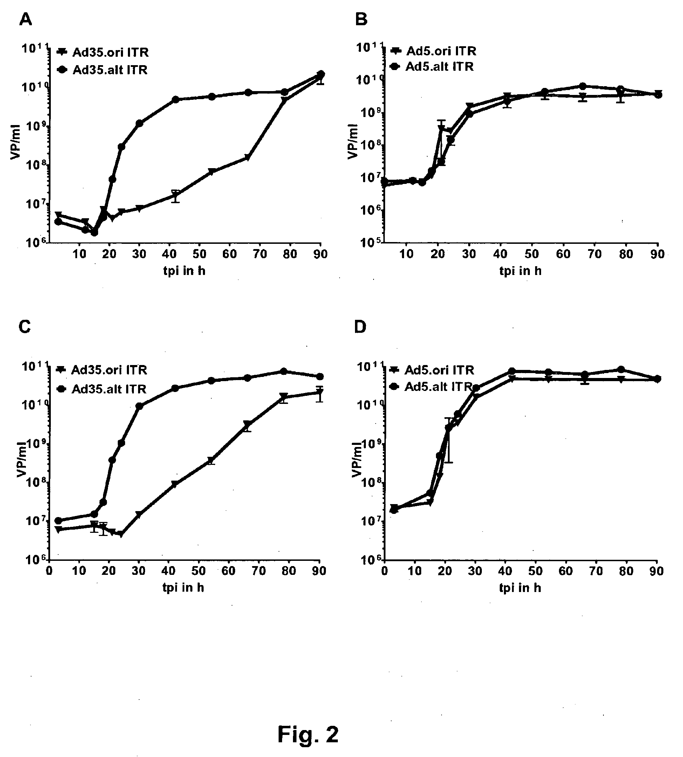

To be able to quantify the suspected difference in replication kinetics, we took advantage of qPCR analysis to measure genome replication at different time points post infection. More specifically, 293 cells were infected at 1000 VP/cell, lysed and subjected to qPCR analysis using a TaqMan assay detecting the CMV promoter, present in the viral vector. As shown in Fig. 2, while both Ad35.ori ITR and Ad35.alt ITR grow to the same titer of approximately 10¹⁰ VP/ml at the latest measured time point (90 hpi) Ad35.ori ITR shows a delayed growth. At early time points post infection, Ad35.alt ITR displays a steeper genome amplification curve, reaching the plateau phase earlier than Ad35.ori ITR (Fig. 2A). In contrast, replication kinetics of Ad5, do not differ for viruses harboring alternative or original ITRs (Fig. 2B).

This genome replication advantage, that is observed for Ad35.alt ITR, but not for Ad5.alt ITR was corroborated on PER.C6 cells (Fig. 2C-D), on which outgrowth of the alternative genome version was originally observed.

### Example 6. The alternative ITR sequence is represented in published human adenovirus sequences

We analyzed whether the alternative ITR sequence was also present in published adenovirus sequences. Thereto, an alignment of nucleotides 1-8 of published human and nonhuman ITRs was performed. Human viruses predominantly harbor the original CATCATCA sequence and were consequently categorized as "conserved human sequences" (Table I).

Additionally, sequences differing from CATCATCA in one to six nucleotides were identified and termed "variable human adenovirus sequences". The predominant sequence among the "variable sequences" was the alternative sequence CTATCTAT that we also identified by passaging Ad35 derived vectors. The alignment of the nonhuman sequences (Table I) shows that CATCATCA is the most frequent sequence. Again, alternative sequences are found, e.g. the previously identified alternative sequence GATGATGT, which is found in fowl adenoviruses. The majority of the published ITR sequences are consistent with the replication model by de Jong (de Jong et al., 2003, Curr Top Microbiol Immunol 272: 187-211; King & van der Vliet, 1994, EMBO J 13: 5786-5792), with a small, two, three or four nucleotide direct repeat that is required for the jumping back mechanism during replication initiation.

It is noted, that Table I shows published ITR sequences that may not be a balanced representation of naturally occurring ITR sequences. In some cases, the terminal nucleotides of the ITRs have not been sequenced but simply inferred to be CATCATCA. Additionally, prior to sequencing growing of adenoviruses from diagnostic swaps is common involving several replication cycles that could result in nucleotide changes. Nevertheless, it is noted that the original sequence CATCATCA is still detected in nature after a long period of virus host co-evolution, and hence the alternative sequence CTATCTAT may be more beneficial in cell culture than in nature.

### Example 7. Repeated passaging leads to a switch in ITR sequence on a variety of cell lines.

To rule out that the observed switch from original to alternative ITRs is a phenomenon restricted to the production E1-complementing cell line, we passaged Ad35.wt virus containing the original ITR sequence CATCATCA on a variety of cell types. Therefore Ad35wt was rescued using plasmids containing the complete Ad35 wild type genome on A549, HEK293, PER.C6, Hep2, HeLa and MRC5 cells. The specific cell lines were chosen to present a broad variety of cell types, including cell lines derived from different tissues, of carcinoma and non-carcinoma origin, epithelial and fibroblastic cell lines and different ploidities (Table III).

The results in Table III show that the switch to alternative ITRs is observed at VPN 10 for the helper cell lines HEK293 and PER.C6 cells, but a switch or a mixing phenotype is also observed for the other tested cell lines, albeit at a later passage number.

### Example 8. Extended passaging induces ITR heterogeneity or a complete shift to the alternative ITR sequence in the majority of the tested Adenovirus vectors.

The generality of switching to the alternative CTATCTAT sequence for adenoviral vectors based upon various serotypes was investigated. Hereto, we passaged Ad26, Ad48, Ad49, Ad11(a), Ad50 and Ad5 derived adenoviral vectors on PER.C6 cells. Viral vectors were passaged until VPN 15 after plaque purification and analyzed by sequencing at VPN 10 and VPN 15. Two different transgenes were included for each vector serotype to additionally exclude an effect of a different transgene.

The results of this set of experiments are shown in Table IV. Surprisingly, we found that all tested vectors except Ad48 switch to the alternative ITR sequence or display a mixing phenotype suggesting that they would convert at a later viral passage number. In line with what we previously observed for Ad5, the original ITR sequence was remained at VPN 10, however started to mix at VPN 15. In contrast, Ad48 derived vectors were the only ones to retain the original ITR sequence up to VPN 15.

However, to stay on the safe side, we suggest to equip all recombinant adenoviruses, including the ones based on Ad5 or even Ad48, with the alternative ITR sequences according to the invention, in order to prevent potential batch heterogeneity due to mutations at the genome ends during culturing of large volumes or after extended passaging. This will ensure that batches of recombinant adenovirus are obtained wherein the genomes of essentially all adenovirus particles comprise the 5' terminal sequence CTATCTAT according to the invention. Moreover, the rescuing of adenoviral vectors harboring this alternative ITR sequence may accelerate production of vaccine vectors.

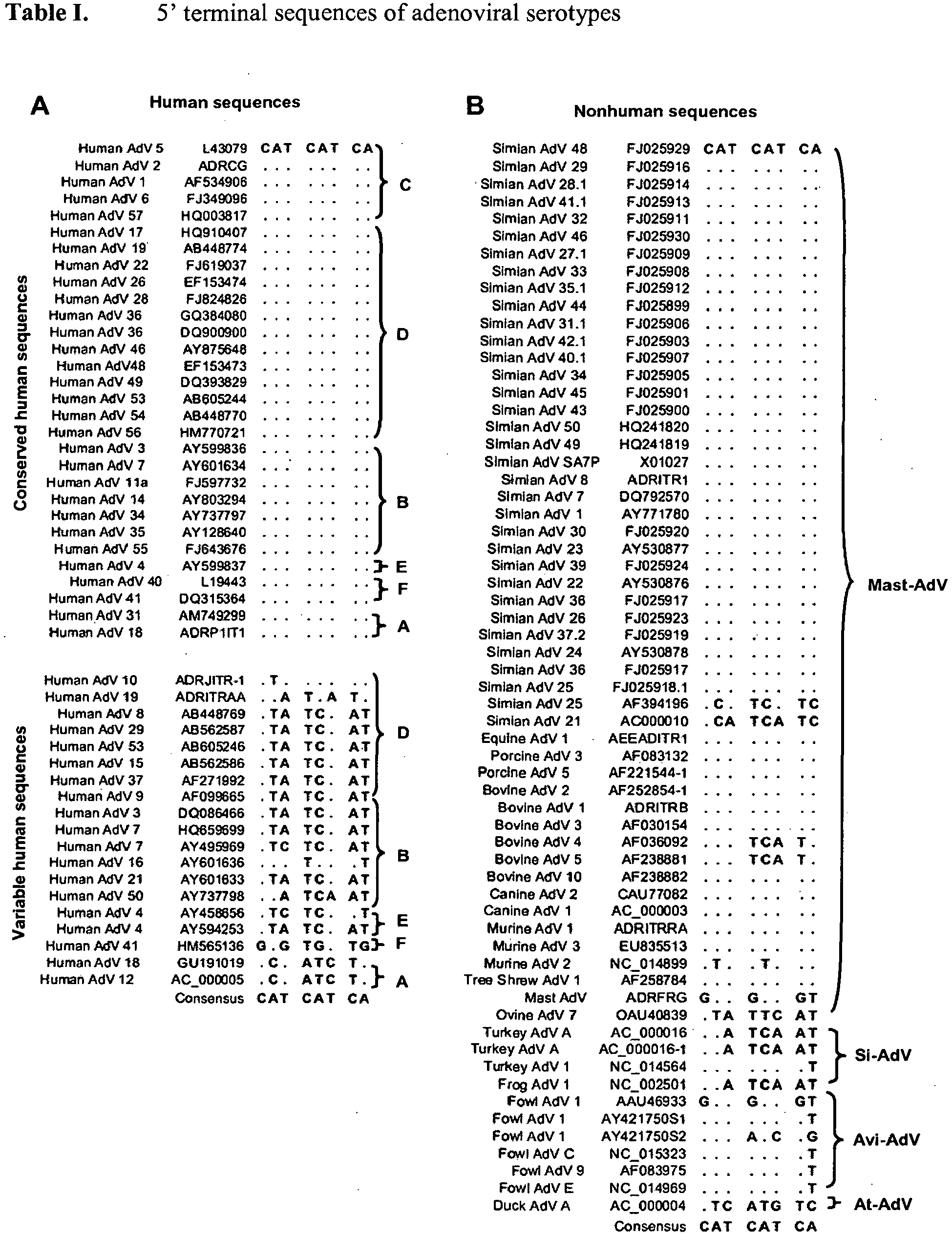

**Table II. ITRs of various rAd35 viruses upon passaging**

| **Virus** | **pIX promoter** | **Genome size (kbp)** | **# PP** | **ITR** |
|---|---|---|---|---|
| **Ad35.TBS** | + | **32.4** | **2** | **Mixing** |
| **Ad35**.**Ebo**.**GP**.**Z** | + | **32.4** | **2** | **alternative** |
| **Ad35**.**Ebo**.**GP**.**S**/**G** | + | **32.4** | **2** | **Mixed** |
| **Ad35.CS** | + | **31.5** | **1** | **Mixed** |
| **Ad35**.**CS** | - | **31.3** | 1 | **original** |
| **Ad35.Luc** | + | **32.0** | 1 | **Mixing** |
| **Ad35.Luc** | - | **31.9** | 1 | **Mixed** |
| **Ad35.eGFP** | + | **31.1** | 1 | **Mixing** |
| **Ad35.eGFP** | - | **30.9** | 1 | **Mixed** |
| **Ad35.Empty** | + | **30.4** | 1 | **alternative** |
| **Ad35.Empty** | - | **30.2** | 1 | **alternative** |
| **Ad35**.**SIV**-**Gag** | + | **31.9** | 1 | **alternative** |
| **Ad35 wildtype** | **NA** | **34.8** | 1 | **Mixed** |

**Table III. ITR switch on different cell lines**

| **Ad35wt** | **Cell type** | **origin** | **ploidity** | **VP10** | **VP15** |
|---|---|---|---|---|---|
| **HEK293** | Helper E1, kidney | epithelial | diploid | alternative | - |
| **PER.C6** | Helper E1, retina | epithelial | hypotriploid | alternative | - |
| **A549** | Lung carcinoma | epithelial | hypotriploid | mixing | Mixing |
| **HeLa** | Cervix adenocarcinoma | epithelial | hypotriploid | - | alternative |
| **Hep2** | HeLa contaminant | epithelial | diploid | original | Mixing |
| **MRC5** | Normal lung | fibroblast | diploid | - | mixing |

**Table IV. ITR switch of different vectors**

| **Vector** | **Subgroup** | **VPN 10** | **VPN 15** |
|---|---|---|---|
| **Ad26.eGFP** | D | mixed | Alternative |
| **Ad26.Luc** | D | mixed | Alternative |
| **Ad48.eGFP** | D | original | Original |
| **Ad48.Luc** | D | original | Original |
| **Ad49.eGFP** | D | alternative | Nd |
| **Ad49.Luc** | D | mixed | alternative |
| **Ad11.En** | B | alternative | Nd |
| **Ad11.SivGag** | B | alternative | Nd |
| **Ad50.eGFP** | B | alternative | Nd |
| **Ad50.Luc** | B | mixed | alternative |
| **Ad5.eGFP** | C | original | Mixing |
| **Ad5.Luc** | C | original | Mixing |

## Claims

1. A composition comprising recombinant adenovirus particles, wherein the recombinant adenovirus comprises a transgene and is a recombinant human adenovirus of serotype 5, 11a, 26, 34,35,49 or 50, or a recombinant simian adenovirus, **characterized in that** the genomes of at least 99% of the adenovirus particles in said composition have as the 5' terminal nucleotides the nucleotide sequence: CTATCTAT.

2. A composition according to claim 1, wherein the recombinant adenovirus is a recombinant human adenovirus of serotype 5, 26, 35, 49 or 50.

3. A composition according to claim 2, wherein the recombinant adenovirus is a recombinant human adenovirus of serotype 26 or 35.

4. A composition according to any one of the preceding claims, which is a pharmaceutical composition.

5. A composition according to any one of the preceding claims, wherein the recombinant adenovirus lacks at least a portion of the E1 region.

6. A composition according to any one of the preceding claims, comprising at least 1 x 10⁷, preferably at least 1 x 10⁸, preferably at least 1 x 10⁹, preferably at least 1 x 10¹⁰ recombinant adenovirus particles.

7. A method for preparing a batch of recombinant adenovirus particles wherein at least 99% of the adenovirus particles in the batch have identical nucleotide sequences in the 5' termini of their genomes and wherein the adenovirus comprises a transgene, the method comprising:
a) performing a molecular cloning step to exchange the naturally occurring 5' termini that are not CTATCTAT of an adenovirus genome with altered 5' termini comprising as the terminal nucleotides the nucleotide sequence: CTATCTAT, .
b) propagating in host cells the recombinant adenovirus having the altered 5' termini, and
c) harvesting the recombinant adenovirus to obtain a batch of reconnbinant adenovirus particles wherein at least 99% of the adenovirus particles in the batch have as the 5' terminal nucleotides of their genomes the nucleotide sequence: CTATCTAT.

8. A method for preparing a batch of recombinant adenovirus particles wherein at least 99% of the adenovirus particles in the batch have identical nucleotide sequences in the 5' termini of their genomes and wherein the adenovirus comprises a transgene, the method comprising:
a) performing a plaque purification of an adenovirus, wherein the recombinant Adenovirus is a recombinant human adenovirus of serotype 5, 11a, 26, 34, 35, 49 or 50, or a recombinant simian adenovirus, to isolate an adenovirus or recombinant adenovirus from a single plaque, wherein said adenovirus or recombinant adenovirus has as the 5' terminal nucleotides of its genome the nucleotide sequence: CTATCTAT,
b) propagating in host cells a recombinant adenovirus obtained from the single plaque of step a), and
c) harvesting the recombinant adenovirus to obtain a batch of recombinant adenovirus particles wherein at least 99% of the adenovirus particles in the batch have as the 5' terminal nucleotides of their genomes the nucleotide sequence: CTATCTAT.

9. A method according to claim 7 or 8, wherein the batch comprises at least 1x10⁷ recombinant adenovirus particles.

10. A method according to any one of claims 7 to 9, wherein the recombinant adenovirus is a recombinant human adenovirus of serotype 5,26,35,49 or 50.

11. A method according to any one of claims 7 to 10, wherein the recombinant adenovirus is a recombinant human adenovirus of serotype 26 or 35.

12. A method according to any one of claims 7 to 11, wherein the recombinant adenovirus lacks at least a portion of the E1 region.

13. A method according to any one of claims 7 to 12, further comprising purifying the recombinant adenovirus.

14. A method according to claim 13, further comprising formulating the recombinant adenovirus into a pharmaceutical composition.

15. A method according to any one of claims 7-14, wherein step b) is performed in a bioreactor.

## Patentansprüche

1. Zusammensetzung, umfassend rekombinante Adenoviruspartikel, wobei das rekombinante Adenovirus ein Transgen umfasst und ein rekombinantes menschliches Adenovirus vom Serotyp 5, 11a, 26, 34, 35, 49 oder 50 oder ein rekombinantes Affen-Adenovirus ist, **dadurch gekennzeichnet, dass** die Genome von wenigstens 99% der Adenoviruspartikel in der Zusammensetzung als 5'-terminale Nukleotide die Nukleotidsequenz: CTATCTAT aufweisen.

2. Zusammensetzung nach Anspruch 1, wobei es sich bei dem rekombinanten Adenovirus um ein rekombinantes menschliches Adenovirus vom Serotyp 5, 26, 35, 49 oder 50 handelt.

3. Zusammensetzung nach Anspruch 2, wobei es sich bei dem rekombinanten Adenovirus um ein rekombinantes menschliches Adenovirus vom Serotyp 26 oder 35 handelt.

4. Zusammensetzung nach einem der vorhergehenden Ansprüche, bei der es sich um eine pharmazeutische Zusammensetzung handelt.

5. Zusammensetzung nach einem der vorhergehenden Ansprüche, wobei dem rekombinanten Adenovirus wenigstens ein Teil der El-Region fehlt.

6. Zusammensetzung nach einem der vorhergehenden Ansprüche, umfassend wenigstens 1 x 10⁷, vorzugsweise wenigstens 1 x 10⁸, vorzugsweise wenigstens 1 x 10⁹, vorzugsweise wenigstens 1 x 10¹⁰ rekombinante Adenoviruspartikel.

7. Verfahren zur Herstellung einer Charge rekombinanter Adenoviruspartikel, wobei wenigstens 99% der Adenoviruspartikel in der Charge identische Nukleotidsequenzen in den 5'-Termini ihrer Genome aufweisen und wobei das Adenovirus ein Transgen umfasst, wobei das Verfahren Folgendes umfasst:
a) Durchführen eines molekularen Klonierungsschritts zum Austauschen der natürlich vorkommenden 5'-Termini, bei denen es sich nicht um CTATCTAT eines Adenovirusgenoms handelt, gegen veränderte 5'-Termini, die als terminale Nukleotide die Nukleotidsequenz: CTATCTAT umfassen,
b) Propagieren des rekombinanten Adenovirus mit den veränderten 5'-Termini in Wirtszellen und
c) Ernten des rekombinanten Adenovirus, so dass eine Charge rekombinanter Adenoviruspartikel erhalten wird, wobei wenigstens 99% der Adenoviruspartikel in der Charge als 5'-terminale Nukleotide ihrer Genome die Nukleotidsequenz: CTATCTAT aufweisen.

8. Verfahren zur Herstellung einer Charge rekombinanter Adenoviruspartikel, wobei wenigstens 99% der Adenoviruspartikel in der Charge identische Nukleotidsequenzen in den 5'-Termini ihrer Genome aufweisen und wobei das Adenovirus ein Transgen umfasst, wobei das Verfahren Folgendes umfasst:
a) Durchführen einer Plaque-Aufreinigung eines Adenovirus, wobei es sich bei dem rekombinanten Adenovirus um ein rekombinantes menschliches Adenovirus vom Serotyp 5, 11a, 26, 34, 35, 49 oder 50 oder ein rekombinantes Affen-Adenovirus handelt, zum Isolieren eines Adenovirus oder rekombinanten Adenovirus aus einem Einzelplaque, wobei das Adenovirus bzw. rekombinante Adenovirus als 5'-terminale Nukleotide seines Genoms die Nukleotidsequenz: CTATCTAT aufweist,
b) Propagieren eines aus dem Einzelplaque aus Schritt a) erhaltenen rekombinanten Adenovirus in Wirtszellen und
c) Ernten des rekombinanten Adenovirus, so dass eine Charge rekombinanter Adenoviruspartikel erhalten wird, wobei wenigstens 99% der Adenoviruspartikel in der Charge als 5'-terminale Nukleotide ihrer Genome die Nukleotidsequenz: CTATCTAT aufweisen.

9. Verfahren nach Anspruch 7 oder 8, wobei die Charge wenigstens 1 x 10⁷ rekombinante Adenoviruspartikel umfasst.

10. Verfahren nach einem der Ansprüche 7 bis 9, wobei es sich bei dem rekombinanten Adenovirus um ein rekombinantes menschliches Adenovirus vom Serotyp 5, 26, 35, 49 oder 50 handelt.

11. Verfahren nach einem der Ansprüche 7 bis 10, wobei es sich bei dem rekombinanten Adenovirus um ein rekombinantes menschliches Adenovirus vom Serotyp 26 oder 35 handelt.

12. Verfahren nach einem der Ansprüche 7 bis 11, wobei dem rekombinanten Adenovirus wenigstens ein Teil der El-Region fehlt.

13. Verfahren nach einem der Ansprüche 7 bis 12, ferner umfassend Aufreinigen des rekombinanten Adenovirus.

14. Verfahren nach Anspruch 13, ferner umfassend Formulieren des rekombinanten Adenovirus in eine pharmazeutische Zusammensetzung.

15. Verfahren nach einem der Ansprüche 7 bis 14, wobei Schritt b) in einem Bioreaktor durchgeführt wird.

## Revendications

1. Composition comprenant des particules d'adénovirus recombiné, dans laquelle l'adénovirus recombiné comprend un transgène et est un adénovirus humain recombiné de sérotype 5, 11a, 26, 34, 35, 49 ou 50 ou un adénovirus simien recombiné, **caractérisée en ce que** les génomes d'au moins 99 % des particules d'adénovirus dans ladite composition ont comme nucléotides d'extrémité 5' la séquence nucléotidique : CTATCTAT.

2. Composition selon la revendication 1, dans laquelle l'adénovirus recombiné est un adénovirus humain recombiné de sérotype 5, 26, 35, 49 ou 50.

3. Composition selon la revendication 2, dans laquelle l'adénovirus recombiné est un adénovirus humain recombiné de sérotype 26 ou 35.

4. Composition selon l'une quelconque des revendications précédentes, qui est une composition pharmaceutique.

5. Composition selon l'une quelconque des revendications précédentes, dans laquelle l'adénovirus recombiné est privé d' au moins une partie de la région E1.

6. Composition selon l'une quelconque des revendications précédentes, comprenant au moins 1 x 10⁷, de préférence au moins 1 x 10⁸, de préférence au moins 1 x 10⁹, de préférence au moins 1 x 10¹⁰ particules d'adénovirus recombiné.

7. Procédé pour la préparation d'un lot de particules d'adénovirus recombiné dans lequel au moins 99 % des particules d'adénovirus dans le lot ont des séquences nucléotidiques identiques aux extrémités 5' de leurs génomes et dans lequel l'adénovirus comprend un transgène, le procédé comprenant :
a) la mise en oeuvre d'une étape de clonage moléculaire pour remplacer les extrémités 5' d'origine naturelle qui ne sont pas CTATCTAT d'un génome d'adénovirus par des extrémités 5' modifiées comprenant en tant que nucléotides terminaux la séquence nucléotidique : CTATCTAT,
b) la propagation dans des cellules hôtes de l'adénovirus recombiné ayant les extrémités 5' modifiées et
c) la récolte de l'adénovirus recombiné pour obtenir un lot de particules d'adénovirus recombiné, au moins 99 % des particules d'adénovirus dans le lot ayant en tant que nucléotides d'extrémité 5' de leurs génomes la séquence nucléotidique : CTATCTAT.

8. Procédé pour la préparation d'un lot de particules d'adénovirus recombiné dans lequel au moins 99 % des particules d'adénovirus dans le lot ont des séquences nucléotidiques identiques aux extrémités 5' de leurs génomes et dans lequel l'adénovirus comprend un transgène, le procédé comprenant :
a) la mise en oeuvre d'une purification par plages d'un adénovirus, l'adénovirus recombiné étant un adénovirus humain recombiné de sérotype 5, 11a, 26, 34, 35, 49 ou 50 ou un adénovirus simien recombiné, pour isoler un adénovirus ou adénovirus recombiné à partir d'une seule plage, ledit adénovirus ou adénovirus recombiné ayant en tant que nucléotides d'extrémité 5' de son génome la séquence nucléotidique : CTATCTAT,
b) la propagation dans des cellules hôtes d'un adénovirus recombiné obtenu à partir de l'unique plage de l'étape a) et
c) la récolte de l'adénovirus recombiné pour obtenir un lot de particules d'adénovirus recombiné, au moins 99 % des particules d'adénovirus dans le lot ayant en tant que nucléotides d'extrémité 5' de leurs génomes la séquence nucléotidique : CTATCTAT.

9. Procédé selon la revendication 7 ou 8, dans lequel le lot comprend au moins 1 x 10⁷ particules d'adénovirus recombiné.

10. Procédé selon l'une quelconque des revendications 7 à 9, dans lequel l'adénovirus recombiné est un adénovirus humain recombiné de sérotype 5, 26, 35, 49 ou 50.

11. Procédé selon l'une quelconque des revendications 7 à 10, dans lequel l'adénovirus recombiné est un adénovirus humain recombiné de sérotype 26 ou 35.

12. Procédé selon l'une quelconque des revendications 7 à 11, dans lequel l'adénovirus recombiné est privé d'au moins une partie de la région E1.

13. Procédé selon l'une quelconque des revendications 7 à 12, comprenant en outre la purification de l'adénovirus recombiné.

14. Procédé selon la revendication 13, comprenant en outre la formulation de l'adénovirus recombiné en une composition pharmaceutique.

15. Procédé selon l'une quelconque des revendications 7-14, dans lequel l'étape b) est mise en oeuvre dans un bioréacteur.
